# EUROPEAN PATENT APPLICATION

(11) **EP 0 832 612 A1**
(43) Date of publication of application: **01.04.1998**
(21) Application number: 97306850.5
(22) Date of filing: 04.09.1997
(51) Int. Cl.: A61B 17/39

(54) **Electrosurgery apparatus**

(30) Priority: 27.09.1996 GB 9620127
(71) Applicant: Smiths Industries Public Limited Company, London, NW11 8DS (GB)
(72) Inventor: Hay, Stewart James John, Worthing, Sussex BN14 8PG (GB)
(74) Representative: Flint, Jonathan McNeill

(57) **Abstract**

Electrosurgery apparatus has a MOSFET switch 17 and 18 connected between ground 16 and each opposite end of the primary winding 15 of an output transformer 11. The centre tap 25 of the primary 15 is connected to a source 26 of dc voltage via a modulating transistor 28 and a shunt switch 29. The MOSFET switches 17 and 18 are switched on and off in antiphase with one another by a gate logic circuit 20, which also controls the signal supplied to the gate of the modulating transistor 28 to produce a full wave rectified voltage at the centre tap 25. This produces a sine wave output on the secondary windings 10 of the transformer 11. When the shunt switch 29 is closed, the modulating transistor 28 is bridged and a dc signal is applied to the centre tap 25, producing a square wave output on the secondary windings 10.

## Description

This invention relates to electrosurgery apparatus of the kind including an output transformer having a primary winding and a secondary winding.

Electrosurgery, or diathermy, employs high frequency electrical currents to produce cutting, coagulation, or a combination of both cutting and coagulation of patient tissue. The effect produced depends largely on the nature of the pulses applied to the electrosurgery electrode. Where the apparatus must be capable of producing both a square wave and a sine wave output, this is achieved by two separate circuits and by connecting the electrode to the desired circuit. This is a disadvantage because it increases the cost, complexity, size and weight of the apparatus.

It is an object of the present invention to provide improved electrosurgery apparatus.

According to one aspect of the present invention there is provided electrosurgery apparatus of the above-specified kind, characterised in that the apparatus includes first and second switches connected between ground and respective opposite ends of the primary winding, a gating circuit for alternately gating the first and second switches so that one switch is closed while the other switch is open, and a drive circuit connected to a centre tap of the primary winding, and that the drive circuit is selectable to supply either a steady voltage to the centre tap when a square wave output is desired across the secondary winding or an alternating sine wave when a sine wave output is desired across the secondary winding.

The first and second switches may include a MOSFET transistor. The drive circuit preferably includes a dc voltage source connected to the centre tap via a modulating device, and a sine wave generator connected with the modulating device. The sine wave generator may be driven by the gating circuit. The sine wave generator preferably includes a second transformer having a primary winding connected with a capacitance to provide a resonant LC circuit. Opposite ends of the primary winding of the second transformer are preferably connected to ground via respective capacitors, a centre tap of the primary winding being connected to ground via a third switch, the third switch being controlled by an output from the gating circuit. Opposite ends of a secondary winding of the second transformer are preferably connected to ground via respective unidirectional current devices, a centre tap of the secondary winding being connected to provide the drive for the modulating device.

Electrosurgery apparatus according to the present invention will now be described, by way of example, with reference to the accompanying drawings, in which:
- Figure 1: is a circuit diagram of the apparatus; and
- Figure 2: shows waveforms at different locations in the apparatus.

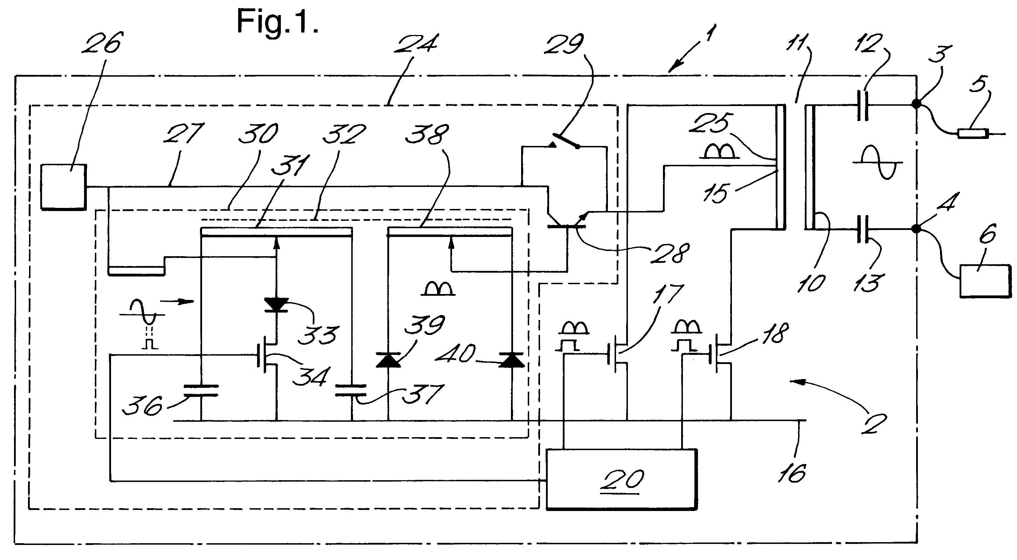

With reference first to Figure 1, the apparatus is indicated generally by the numeral 1 and includes a waveform generator 2. The apparatus 1 also includes various other conventional features, such as for power control, safety and monitoring, which are not shown because they are not relevant for an understanding of the present invention. The apparatus 1 has output terminals 3 and 4 connected respectively to an active hand-held electrode 5 and a large-area plate, return electrode 6.

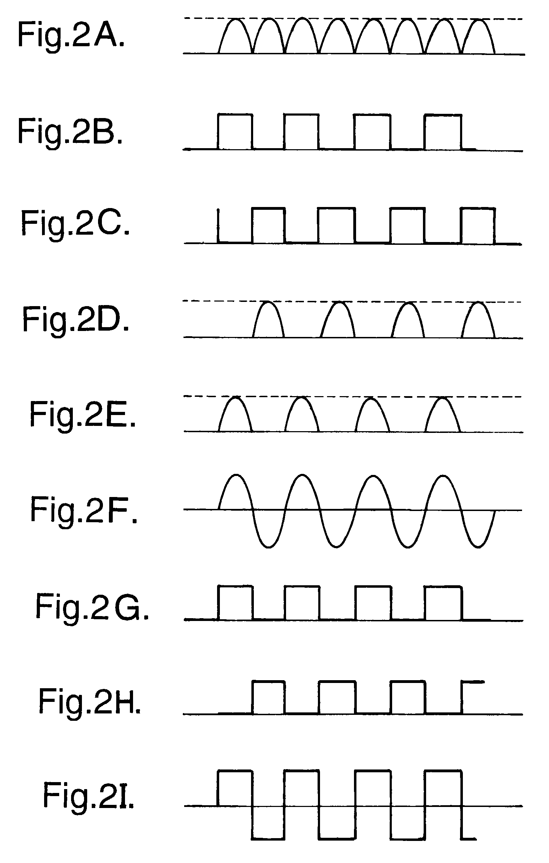

The two electrodes 5 and 6 are connected to opposite ends of a secondary winding 10 of an output transformer 11, via respective capacitors 12 and 13. The transformer 11 has a primary winding 15 with opposite ends connected to a ground rail 16 via respective MOSFET transistor switches 17 and 18, the drains of the transistors being connected to the winding. The waveform generator 2 includes a gate logic circuit 20 connected to the gates of the transistors 17 and 18. The logic circuit 20 produces two identical square-wave outputs of the kind shown in Figures 2B and 2C, the two outputs being 180° out of phase with each other so that one output is high while the other is low. The high period of each pulse is slightly longer than the low period so that there is a short dead time between the high periods of the two sets of pulses. The logic circuit 20 switches the transistors 17 and 18 on and off alternately, with one transistor being non-conductive while the other is conductive.

The waveform generator 2 also includes a drive circuit 24 connected to the centre tap 25 of the primary winding 15. The drive circuit 24 includes a dc voltage supply 26 producing a dc voltage of about 100 volts on a supply line 27 connected to the centre tap 25. The supply line 27 has a bipolar modulating transistor 28 connected with its collector and emitter in series in the supply line, and a manually-operated switch 29 connected in parallel across the transistor. The base of the transistor 28 is connected to a sine wave generator 30 producing a full-wave rectified sine wave voltage. The generator 30 has a resonant LC circuit where the inductance is provided by the primary winding 31 of a transformer 32 constructed to store an appreciable amount of energy. The centre tap of the primary winding 31 is connected to both the dc voltage supply 26 and to the ground rail 16, via a series connection of a diode 33 and a MOSFET transistor 34. The gate of the transistor 34 is connected to an output of the logic circuit 20, which produces square wave pulses at the desired operating frequency of the waveform generator, typically about 350 kHz. The two opposite ends of the primary winding 31 are connected to the ground rail via respective capacitors 36 and 37 providing the capacitive element of the resonant LC circuit. The secondary winding 38 of the transformer 32 has its centre tapping connected to the base of the modulating transistor 28. Opposite ends of the secondary winding 38 are connected to the ground rail 16 via respective diodes 39 and 40.

The operation of the apparatus will now be described also with reference to Figure 2.

The pulse input from the logic circuit 20 switches the transistor 34 on and off at the resonant frequency of the LC circuit formed by the primary winding 31 of the transformer 30 and the two capacitors 36 and 37. This produces an alternating sine wave across the primary winding 31. The output at the centre tap of the secondary winding 38 is, therefore, a sine signal full-wave rectified by the diodes 39 and 40. This signal is supplied to the base of the bipolar transistor 28 so that the voltage on the supply rail 27 is modulated accordingly and a full-wave, rectified voltage, of the kind shown in Figure 2A, is supplied to the centre tap 25 of the primary winding 15 of the output transformer 11. The transistors 17 and 18 connected to opposite ends of the primary winding 15 are switched by their inputs shown in Figures 2B and 2C respectively, in synchronism with the alternating signal at the centre tap 25. The signals at the drains of the two transistors 17 and 18 are, therefore, alternating sine wave signals of the kind shown in Figures 2D and 2E respectively. This produces a sine wave output on the secondary winding 10 of the transformer 11 of the kind shown in Figure 2F.

To produce a square wave output instead, the user closes the manual switch 29, so that the transistor 28 is shunted and the centre tap 25 of the primary winding 15 receives a steady dc voltage. The signals at the drains of the two transistors 17 and 18 are then square waves of the kind shown in Figures 2G and 2H respectively. The output on the secondary winding 10 is then an alternating square wave of the kind shown in Figure 21.

The apparatus can, therefore, easily be switched between a sine wave and a square wave output whilst still using the same output transformer.

## Claims

1. Electrosurgery apparatus including an output transformer (11) having a primary winding (15) and a secondary winding (10), characterised in that the apparatus includes first and second switches (17 and 18) connected between ground (16) and respective opposite ends of the primary winding (15), a gating circuit (20) for alternately gating the first and second switches (17 and 18) so that one switch is closed while the other switch is open, and a drive circuit (24) connected to a centre tap (25) of the primary winding (15), and that the drive circuit (24) is selectable to supply either a steady voltage to the centre tap (25) when a square wave output is desired across the secondary winding (10) or an alternating sine wave when a sine wave output is desired across the secondary winding (10).

2. Electrosurgery apparatus according to Claim 1, characterised in that the first and second switches include a MOSFET transistor (17, 18).

3. Electrosurgery apparatus according to Claim 1 or 2, characterised in that the drive circuit (24) includes a dc voltage source (26) connected to the centre tap (25) via a modulating device (28), and a sine wave generator (30) connected with the modulating device (28).

4. Electrosurgery apparatus according to Claim 3, characterised in that the sine wave generator (30) is driven by the gating circuit (20).

5. Electrosurgery apparatus according to Claim 3 or 4, characterised in that the sine wave generator (30) includes a second transformer (32) having a primary winding (31) connected with a capacitance (36, 37) to provide a resonant LC circuit.

6. Electrosurgery apparatus according to Claim 5, characterised in that opposite ends of the primary winding (31) of the second transformer (32) are connected to ground (16) via respective capacitors (36 and 37), that a centre tap of the primary winding (31) is connected to ground (16) via a third switch (34), and that the third switch (34) is controlled by an output from the gating circuit (20).

7. Electrosurgery apparatus according to Claim 5 or 6, characterised in that opposite ends of a secondary winding (38) of the second transformer (32) are connected to ground (16) via respective unidirectional current devices (39 and 40), and that a centre tap of the secondary winding is connected to provide the drive for the modulating device (28).
